# EUROPÄISCHE PATENTANMELDUNG

(11) **EP 0 667 118 A1**
(43) Veröffentlichungstag der Anmeldung: **16.08.1995**
(21) Anmeldenummer: 95101667.4
(22) Anmeldetag: 08.02.1995
(51) Int. Cl.: A61B 10/00

(54) **Vorrichtung zur Temperaturmessung zum Feststellen der Ovulation**

(30) Priorität: 10.02.1994 DE 4404238; 16.03.1994 DE 4408911; 21.04.1994 DE 4413881; 18.11.1994 DE 4441239
(71) Anmelder: Mollen, Herbert, D-53347 Alfter (DE)
(72) Erfinder: Mollen, Herbert, D-53347 Alfter (DE)

(57) **Zusammenfassung**

Anordnung zur Ermöglichung einer fortlaufenden Entnahme subfebriler Körpertemperaturen während der Nachtruhe und im Verlauf des weiblichen Zyklus dadurch gekennzeichnet, daß ein Temperaturfühler, der als eigenstabile Sonde konzipiert ist, in die Vagina eingeschoben und eingelagert werden kann und durch ein flexibles Meßkabel die aufgenommenen Temperaturdaten zur Auswertung nach außen geleitet werden können.

## Beschreibung

Vorrichtung zur laufenden Messung und Auswertung von Körpertemperaturen, speziell der subfebrilen Ruhetemperaturen im Verlauf des weiblichen Zyklus .

Der Bereich der genannten subfebrilen Temperaturen liegt unterhalb des normalen Niveaus der Körpertemperaturen.Diese sind beim gesunden Menschen zwischen 37 und 37,2 Grad Celsius stabilisiert.Subfebrile Temperaturen,also solche, die unterhalb dieses normalen Niveaus liegen,gibt es unter physiologischen Gegebenheiten nur bei geschlechtsreifen Frauen und während des Verlaufs ihres normalen hormonalen Zyklus.

Diese subfebrilen Temperaturen und ihr besonderer Verlauf werden bestimmt einerseits durch das ständig aus dem Eierstock der Frau ausgeschiedene sogenannte Follikelhormon,das eine die Temperatur absenkende Einwirkung auf das Wärme - zentrum im Gehirn hat,und anderseits durch das sogenannte Gelbkörperhormon,das einen temperatursteigernden Effekt auf das Wärmezentrum ausübt Das Gelbkörperhormon bildet sich in der zweiten Hälfte des Zyklus nach dem Platzen des gereiften Eifollikels aus einer kleinen Drüse,der sogenannten Gelbkörperdrüse,die sich jedesmal neu aus der Wand des geplatzten Follikels entwickelt.

Unter üblichen Voraussetzungen ist eine auswertbare Erfassung dieses subfebrilen Temperaturgeschehens nicht möglich.Die Wärme-produktion des wachaktiven Organismus überlagert völlig diese Temperaturvorgänge.Nur beim Vorliegen einer gänzlichen Abreaktion des Organismus könnte man Höhe und Verlauf der subfebrilen Temperaturwerte klar und auswertbar erfassen.

Bisher hat man sich dazu auf eine Erfassung der morgendlichen Aufwachtemperaturen beschränken müssen also, nachdem während der nächtlichen Ruhephase die Aktivitätswärme des Organismus abgeklungen war .Solche Einzelmessungen ergaben aber kein ausreichendes sicher auswertbares Datenmaterial für eine präzise Ausage über dieses hormonal geprägte subfebrile Temperaturgeschehen.

Es wäre aber äußerst wichtig eine wirkliche Transparenz und damit sichere Auswertungsmöglichkeit in diesen praktisch unbekannten speziellen Körpertemperaturbereich der subfebrilen Temperaturen zu erreichen.Dabei handelt es sich - von vielfältigen sonstigen Anwendungsmöglichkeiten in der Medizin einmal abgesehen - erstrangig um die seit langen einer Löharrenden Sicherung einer natürlichen Familienplanung durch Perfektionierung der bei ihr in wachsendem Umfange angewandten sogenannten Temperaturmeßmethode.

Die Grundlagen dieser Methode zur Abgrenzung der empfängnisfreien Tage im Zyklus der Frau sind allgemein bekannt.Wie schon erwähnt wurde,entsteht nach dem Platzen des Eifollikels und der Ausstoßung des reifen Eies aus der Wand des geplatzten Eifollikels die Gelbkörperdrüse,die einen temperatursteigernden Einfluß auf das Wärmezentrum im Gehirn ausübt.Da aber die ausgestoßene reife Eizelle nur wenige Stunden befruchtungsfähig bleibt,würde eine sichere Methode festzustellen,ob die in der zyklusrelevanten Zeit nach dem Eisprung gemessenen subfebrilen Temperaturvorgänge zweifelsfrei vom Gelbkörperhormon hervorgerufen wurden,bedeuten,daß mit praktisch absoluter Sicherheit die empfängnisfreie Zeit in der zweiten Hälfte des Frauenzyklus bestimmt werden könnte.

Mit bisher üblichen mehrtägigen Einzelmessungen der morgendlichen Aufwachtemperaturen,mit denen eine bestimmte Temperatursteigerung als Bewertung für die hormonale Genese angesetzt werden sollte,ließ sich aber eine wirklich sichere Erkenntnis nicht erzielen.

Da nur wenige Einzelmessungen ausgewertet werden konnten,war es durchaus möglich,daß auch Temperaturen anderer Genese sich in diesen Zyklusbereich mit äquivalenten Einzelwerten etablieren konnten und so Fehlentscheidungen vorgegeben waren.Dies war dann besonders kritisch,wenn gleichzeitig eine gewisse Hinauszögerung des Eisprungs vorangegangen war,die bekanntlich aus verschiedenartigen Gründen möglich ist.

Es gibt verschiedene Entstehungsursachen für das Auftreten solcher zu Irrtümer Anlaß gebenden Temperaturen.Wahrscheinlich entstehen sie am häufigsten dadurch,daß ganz geringfügige fieberhafte Temperatursteigerungen verschiedenster Genese unter dem Einfluß der temperatursenkenden Einwirkung des Follikelhormons in den subfebrilen Bereich herabgedrückt werden können.Bei einer Herauszögerung des Eisprungs kann die ja weiterlaufende Follikelhormonproduktion mit ihren subfebrilen Temperaturabläufen durchaus im Bewertungsbereich der bisherien Temperaturmeßmethode liegen und zu einem der häufigeren Anlässe für Fehlbeurteilungen werden.

Als Absicherung gegen irrtümliche Entscheidungen müßte daher eine Methode gefunden werden ,durch das Gelbkörperhormon hervorgerufene Temperaturabläufe ausnahmslos sicher gegeüber andere abgrenzen zu können.

Das ist nach den diesseitigen Feststellungen nur dadurch möglich, wenn eine größere Anzahl von Temperaturmessungen in einer Abreaktionsphase des Organismus durchgeführt und dann ausgewertet werden können.

Bei der Frau bietet sich hierzu als optimaler Ort eine Temperaturabnahme in der Vagina während der nächtlichen Ruhephase an Hierzu kann ein Temperaturfühler mit einem anschließenden aus der Vagina hinauführenden flexiblen Meßkabel verwendet werden, der zweckmäßig im Bereich des in der Vagina zu liegen kommenden Teilstückes eigenstabilisiert sein sollte,um das Vorschieben in die Scheide zu erleichtern.Notwendig erscheint es auch den eigenstabilisierten Meßkabelteil mit einem weichen Material zu ummanteln,sodaß Irritationen beim Einführen und Lagern in der Vagina vermieden werden.

Die aufgenommen Temperaturdaten werden über das hinausführende flexible Meßkabel zu einem kleinen vorzüglich aus Kunststoff bestehenden kleinen Gehäuse geführt,in dem die zur Erreichung des Erfindungszweckes vorgesehen Vorrichtungen also Digitalisierung der Temperaturwerte,eine Zeitintervallsetzung,Speicherplätze,die Anordnungen zur Auswertung nebst einer Batterie integriert sind.Das Gehäuse kann außen an einer Körperstelle zum Beispiel an einem Hüftgürtel abnembar fixiert werden.

Eine andere Möglichkeit,die ein herausführendes Kabel unnötig macht,setzt eine Trennung zwischen den größenordnungsmäßig an sich schon kleinen Vorrichtungen bis zur Datenbereitstellung und den größenordnungsmäßig an sich schon umfangreicheren Vorrichtungen zur Auswertung der Daten voraus.Nach dem heutigen Stand der Technik bzw. der konstruktiven Möglichkeiten ließen sich die einzelnen Teile bis zur Datenbereitstellung also Temperaturfühler,Digitalisierungsvorich- tung,Zeitintervallsetzung und Speicherung nebst einer kleinen Batterie so minimieren, daß sie akzeptionsconform in die Vagina deponiert werden können.Zusammengefaßt bräuchten sie nicht wesentlich Größe und Durchmesser eines Menstruationstampons überschreiten.Sie könnten - unter Freilassung des Temperaturfühlers - in einem hülsenartigen Kunststoffgehäuse eingebaut werden.Zweckmäßig wäre es, wenn man das Gehäuse - ebenfalls unter Beachtung einer unbehinderten Temperaturübertragung - in eine Umhüllung aus reißfesten Material einschieben könnte, die zudem außen mit einem weichen Material ummantelt sein könnte,sodaß eine irritationsfreie Lagerung in der Vagina gewährleistet wäre. Mit einer an der reißfesten Umhüllung befestigten Zugvorrichtung -wie etwa beim Menstruationstampon - könnte die Temperatursonde nach Beendigung der Meßphase zur anschließenden Auswertung der gespeicherten Daten der Vagina wieder entnommen werden.

Durch die Zeitintervallsetzung ist die benötigte größere Anzahl auswertbarer Ruhetemperaturdaten in den Speicherplätzen abgelegt worden und können von dort abgerufen werden Zunächst bietet sich eine Auswertung auf der Basis eines optischen Vergleichs der Höhe der abgerufenen Temperaturdaten mit der Höhe der fixierten morgendlichen Aufwachtemperatur an.Diese vergleichende Überprüfung könnte in nebeneinander angeordneten LCD Anzeigen der beiden Temperaturwerte erfolgen. Übersteigen dabei die abgerufenen Temperaturdaten nichthäufiger die Höhe der Aufwachtemperatur ,so kann mit größter Verläßlichkeit vom Vorliegen einer vom Gelbkörperhormon beeinflußten Temperaturabfolge ausgegangen werden und damit von einer entsprechenden Aussage im Sinne der Aufgabenstellung.

Die Abberufung der Daten sollte vom Temperaturwert der letzten Messung - also von der Aufwachtemperatur aus - sozusagen rückwärts erfolgen.

Das typische Kurvenbild eines vom Gelbkörperhormon beeinflußten Temperaturverlaufs kann so bis zu dessen Ende,dies wäre bis zum tiefsten Punkt der Temperaturabfolge,bei dem das völlige Abklingen der Aktivitätswärme des Organismus erreicht wurde,schnell und genau erfaßt und gemäß den vorgegebenen Kriterien ausgewertet werden.

Eine elektronische Auswertung ließe sich unabhängig oder in Kombination mit einer optischen Überprüfung über eine Grenzwertfestsetzung erreichen,die zum Beispiel über eine Taste eingegeben werden könnte.Eine solche Grenzwertfestsetzung könnte sich an die Schwankungsbreite der Temperaturkurve und so im praktischem Falle an die Höhe der vorliegenden Aufwachtemperatur ausrichten.Wird dieser eingestellte Grenzwert überschritten,so wird etwa ein Transistorausgang aktiviert,durch den ein Ereigniszähler induziert wird,der die Häufigkeit der Überschreitungen des Grenzwertes registriert.

Diese Häufigkeitserfassung kann dann als sichere Grundlage einer elektronischen Auswertung dienen.Die Bewertung stützt sich dabei auf die Grundfeststellung,daß eine durch das Gelbkörperhormon geprägte Temperaturabfolge in einem typischen schmalen Temperaturkorridor verläuft,der eine Schwankungsbreite von höchstens o,15 Grad Celsius besitzt.Subfebrile Temperaturvorgänge anderer Entstehungsursache,auf die schon hingewiesen wurde,könnten einen solchen spezifischen Verlauf nicht nachvollziehen.

Diese Temperaturen zeigen aufgrund ihrer völlig anderen Entstehungsursache ein gänzlich unterschiedliches unzusammenhängendes Verlaufsbild mit meist sprunghaften Schwankungen und damit weitgehnder Streung der einzelnen erfaßten Temperaturpunkte.Es erscheint daher praktisch ausgeschlossen, daß eine größere Anzahl dieser gestreuten Temperaturpunkte, aus anderer Entstehungsursache herrührend, sich in dieser schmalen Bandbreite eines vom Gelbkörperhormon geprägten Temperaturverlaufs etablieren könnten.

Somit kann rückschließend davon ausgegangen werden,daß dann, wenn bei einer Überprüfung der abgerufenen Daten keine oder nur eine geringe Anzahl den kennzeichnenden Korridor überschreiten,also eine weitaus überwiegende Anzahl sich innerhalb des engen Temperaturkorridors befinden müssen,mit Sicherheit ein bestimmender Einfluß des Gelbkörperhormons vorliegen muß und damit ein schon erfolgter Eisprung.

Grundsätzlich kann also aus der Anzahl der,wie beschrieben, durch Grenzwertsetzung ermittelten Überschreitungen zur Gesamtzahl der ausgewerteten Daten eine klare Entscheidung im Sinne der Aufgabenstellung getroffen werden.

Zusätzlich kann vorgesehen werden,daß bei Erreichen einer bestimmten Anzahl von Überschreitungen,welche eine hormonale Genese des Temperaturverlaufs in Frage stellen würde,ein Signal als Warnung ausgelöst wird zum Beispiel ein Blinken in der zur Meßstelle gehörenden LED Anzeige.

Es ist bei diesem perfektionierten Temperaturmeßverfahren im allgemeinen nur eine einmalige nächliche Temperaturabnahme innerhalb eines Zyklus in der vorgesehenen Zeit erforderlich.

Dies erhöht natürlich wesentlich die Praktikabilität der Temperaturmeßmethode und damit die Akzeptanz Im Zusamenhang mit der Frage der Akzeptanz sei noch auf folgendes hingewiesen.

Als Alternative zu einer vaginalen Messung wäre es auch möglich die nötige größere Anzahl von Temperaturdaten während der nächtlichen Ruhephase in der Achselhöhle zu entnehmen.Hierbei könnte die kleine Temperatursonde,wie sie bei der vaginalen Messung vorgesehen ist,in die Achselhöhle eingelegt und dort fixiert werden.Diese Fixierung ließe sich durch eine positionsgerecht angebrachte Oberarmmanschette ermöglichen,an deren Innenseite die Sonde in günstiger Meßposition eingeschoben werden kann.Eine Innenausstattung der Manschette mit weichen isolierenden Material kann gewährleisten,daß kein temperaturmindernder Wärmeverlust auftreten kann.

Im allgemeinen ist die Temperaturmessung in der Achselhöhle weniger exakt als in einer Körperhöhle deren gut durchblutete Schleimhäute zu einer schnellen Übertragung der Körperwärme auf den Meßfühler sorgen.Da aber die nächtliche Meßzeit lang genug ist,um eine ausreichende Temperaturübertragung zu sichern und dazu der Wärmeverlust am achsialen Meßort verhindert wird,kommt der Nachteil einer Messung in der Achselhöhle praktisch zum Fortfall.Es kann sich,wenn überhaupt,nur ein ganz geringfügige relativ gleichmäßige Absenkung des Gesamtniveaus des Temperaturverlaufs bemerkbar machen.Dies spielt aber für die Sicherheit der Meßmethode keine Rolle,da es ja nicht auf absolute Celsiusgradhöhe ankommt,sondern auf den charakteristischen Gesamtverlauf der Kurve,der ja nicht beeinträchtigt wird.

Es soll weiter vorgesehen sein,daß die Daten über eine Schnittstelle in eine Diskette oder dierekt in einem Computer eingegeben werden können. Dies interessiert natürlich erstrangig den Arzt,der die Daten dann über Computer und Monitor,die in einer modernen Arztpraxis zur Verfügung stehen,in einem Kurvenbild zur Auswertung darstellen kann.Es ergibt sich daraus für den Arzt Möglichkeiten in der Fertilitätsberatung,zur Diagnose von Zyklusstörungen und begleitende Überprüfung von Behandlungen.

Auch für die Überprüfung von Fieberzuständen könnte der Teil des Erfindungsgedankens in Betracht gezogen werden, der eine separate Zusammenfassung der Funktionsteile von der Temperaturabnahme bis zur Speicherung in einer kleinen Temperatur-sonde vorsieht.Es könnte damit eine bisher fehlende einfache instrumentelle Anordnung zur laufenden Überprüfung und Aufzeichnung von Fiebertemperaturen ermöglicht werden.

In einer zweckentsprechenden flachen Sonde aus Kunststoff,in der die Teile eingebaut sind,könnten die Fiebermessungen in der Achselhöhle durchgeführt werden,wo sich die Sonde durch eine Oberarmmanschette fixieren ließe. Die Innenseite der Manschette könnte zudem mit einer weichen und isolienden Schicht ausgestattet werden.Durch Auswertung der gespeicherten Daten ließe sich jederzeit und laufend eine Überprüfung des Fieberzustandes durchführen.

Bisher ließen sich solche laufenden Kontrollen nur durch meist unzulängliche personal- und zeitaufwendige Einzelmessungen durchführen. Die vorhandenen aparativen Möglichkeiten standen wegen des großen Kostenaufwandes zur Anschaffung solcher größeren Geräte nur in beschränkten Umfange und nur für besondere Einzelfälle zur Verfügung.

Über eine Schnittstelle ließen sich die gespeicherten Daten einem Computer zuführen,der über einen Monitor das Kurvenbild des Temperaturverlaufs zur Darstellung bringen kann.Dieser ließe sich natürlich auch über eine Diskette speichern,von der sie abgerufen werden kann.Über einen Drucker ließe sich die Temperaturkurve auch dokumentarisch festhalten.

Auch in der häuslichen Krankenbetreuung kann die einfache Meßvorichtung zu einer laufenden Kontrolle eingesetzt werden. Die gespeicherten Daten können den behandelnden Arzt überbracht werden,der sie auswerten kann.Es ist hierbei davon auszugehen,daß in einer heutigen Artzpraxis die zur Auswertung benötigten Geräte vorhanden sind.Eine Einzelmessung mit Darstellung über eine LED Anzeige muß natürlich gwährleistet bleiben.

Zweckmäßig wäre es zudem über eine Anschlußbuchse,die sich außerhalb der fixierenden Manschette an leicht zugänglicher Stelle befinden könnte,die gespeicherten Temperaturen beliebig abrufen zu können.Es bräuchte zur Überprüfung des Temperaturgeschehens nicht jedesmal die Manschette abgenommen bzw.die Temperatursonde herausgenommen zu werden. Eine extreme Vereinfachung einer laufenden Temperaturüberwachung wäre so ermöglicht worden.

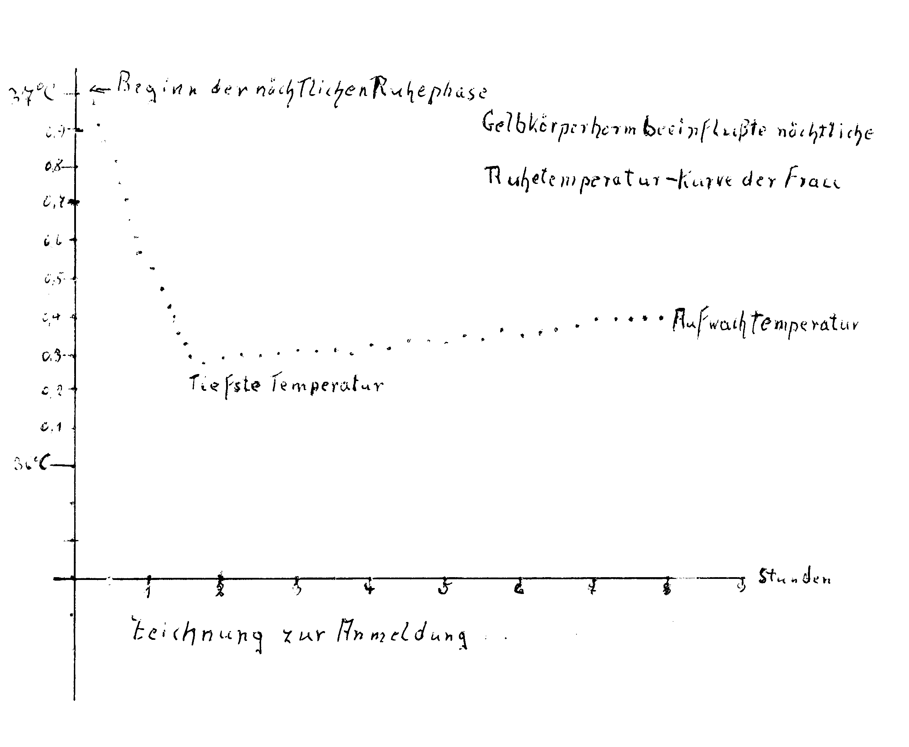

## Patentansprüche

1. Anordnung zur Ermöglichung einer fortlaufenden Entnahme subfebriler Körpertemperaturen während der Nachtruhe und im Verlauf des weiblichen Zyklus dadurch gekennzeichnet,daß ein Temperaturfühler,der als eigenstabile Sonde konzipiert ist, in die Vagina eingeschoben und eingelagert werden kann und durch ein flexibles Meßkabel die aufgenommenen Temperaturdaten zur Auswertung nach außen geleitet werden können.

2. Anordnung zur laufenden Entnahme subfebriler nächtlicher Ruhetemperaturen in der Achselhöhle dadurch gekennzeichnet,daß eine dem Meßort angepaßte z.B. flache Temperatursonde eine sichere effektive Entnahme in der Achselhöhle ermöglicht und daß die Temperatur-sonde durch eine zweckmäßige Haltevorrichtung etwa durch eine positionsgerecht angebrachte Oberarmmanschette fixiert werden kann und daß durch eine Innenauskleidung der Manschette mit einem weichem isolierenden Überzug gewährleistet wird,daß kein temperaturmindernder Wärmeverlust auftreten kann.

3. Anspruch dadurch gekennzeichnet,daß die gemäß 1 u 2 entnommenen subfebrilen Ruhetemperaturen zur Ermöglichung einer speziellen Auswertung im Sinne der erfinderischen Aufgabenstellung durch eine Kombinierung von Digitalisierung,laufender Abfolge mit Zeitintervallsetzung und Speicherung erstmalig eine optimale notwendige Aufbereitung und Aufschließung erhalten

4. Minimierung der Einzelteile der unter 3 beschriebenen Anordnung zur Aufschließung und Aufbereitung der gemessenen subfebrilen Ruhetemperaturen dadurch gekennzeichnet, daß es dadurch ermöglicht werden soll,die minimierten Teile in eine kleine Sonde einzubauen,die dann in die Vagina oder Achselhöhle eingelegt werden kann,dort also die Speicherung der Temperaturdaten vor sich geht und nach Wiederherausnahme der Sonde in der zur Verfügung stehenden Vorrichtung die Auswertung der gespeicherten Daten erfolgen kann.

5. Anordnung dadurch gekennzeichnet,daß die gemäß 1-3 gespeicherten Daten einzeln abberufen und in einer LCD sichtbar gemacht werden können und diese Daten in einer optimalen optischen Vergleichsposition zu einer zweiten LCD Anzeige angeordnet werden soll in der die Aufwachtemperatur fixiert ist.

6. Anordnung dadurch gekennzeichnet,daß zur Klärung der Genese der aufgezeichneten subfebrilen Ruhetemperaturen die abzurufenden Daten der Kontrolle einer Grenzwertfestsetzung unterworfen werden,die an der physiologischen Schwankungspreite eines vom Gelbkörperhormon induzierten subfebrilen Temperaturverlaufs orientiert ist.

7. Anordnung gemäß 6 dadurch gekennzeichnet ,daß der Grenzwert an der Höhe der Aufwachtemperatur des aktuell vorliegenden Meßvorgangs ausgerichtet wird,die etwa durch Drucktaste der Vorrichtung zur Grenzwertkontrolle eingegeben werden kann.

8. Anordnung gemäß 6 u.7 dadurch gekennzeichnet,daß bei einer Überschreitung der Grenzwertfestsetzung ein Transsistorausgang oder entsprechende Vorrichtung aktiviert werden,die einen Ereigniszähler induzieren,der die Anzahl der Temperaturwerte registriert,welche die Grenzwertfestsetzung überschreiten.Aus dem Verhältnis der Anzahl dieser registrierten Überschreitungen zur Gesamtzahl der abgerufenen Daten kann eine klare Entscheidung im Sinne der Augabenstellung erfolgen.Zusätzlich kann bei Überschreiten einer eingegebenen kritischen Zahl der Überschreitungen,welche also die hormonale Genese des Temperaturablaufs in Frage stellen würde,automatisch Signals ausgelöst werden können z.B. ein Blinken in der zur Meßstelle gehörenden LED Anzeige.

9. Anspruch dadurch gekennzeichnet,daß die Anordnung zur laufenden Temperaturabnahme gemäß 1 u. 2 ,die Aufbereitung und Speicherung gemäß 3 und eine Temperatursondengestaltung gemäß 4 zur laufenden Erfassung und Speicherung von Fiebertemperaturen insbesondere bei deren Abnahme in der Achselhöhle,wie unter 2 dargestellt,eingesetzt werden können und die gespeicherten Temperaturen von einer sich außerhalb der Manschette befindlichen Anschlußbuchse abberufen und in einer LCD Darstellung oder durch Überprüfung in sonstigen Vorrichtungen ausgewertet werden können.
